(19)

Europäisches
Patentamt
European
Patent Office
Office européen
des brevets

(11) **EP 2 848 929 A1**

(12) **EUROPEAN PATENT APPLICATION**

(43) Date of publication:
**18.03.2015 Bulletin 2015/12**

(51) Int Cl.:
*G01N 27/414* [(2006.01)]   *G01N 33/543* [(2006.01)]
*G01N 33/00* [(2006.01)]

(21) Application number: **13184003.5**

(22) Date of filing: **11.09.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **AIT Austrian Institute of Technology GmbH**
**1220 Wien (AT)**

(72) Inventor: **Nowak, Christoph**
**1180 Vienna (AT)**

(74) Representative: **Sonn & Partner Patentanwälte**
**Riemergasse 14**
**A-1010 Wien (AT)**

(54) **Graphene FET-based biosensor**

(57)    The present invention relates to a biomolecular sensor device comprising at least one graphene layer as semi-conductor material, wherein said graphene layer comprises bound to its surface a ligand binding protein, with the protein modifying an electrical property of said device, such as impedance or transistor gating of the graphene semi-conductor, especially upon ligand binding and/or conformational change of said protein, said sensor is suitable for detecting odorant molecules capable of binding to olfactory binding proteins or membrane bound receptors. The invention provides the first graphene layer that is area-attached to a lipid bilayer. Further provided are methods of use of the sensor in detecting ligands to the proteins and methods of manufacture.

Fig. 5

EP 2 848 929 A1

**Description**

The present invention relates to the field of electrochemical sensors with proteinaceous selectivity elements.

The olfactory system has the remarkable capacity to discriminate a wide range of odor molecules. In humans, smell is rather considered to be an esthetic sense in contrast to most other species, which rely on olfaction to detect food, predators and mates. In vivo, olfaction occurs when odorant molecules bind to specific sites on the olfactory receptors (ORs) in the olfactory epithelium. A further class of proteins, odorant binding proteins or olfactory binding proteins (OBPs), are assumed to transport odorant molecules to the olfactory epithelium. OBPs bind different odorants as ligands with different affinity. OPs may bind odorants or complexes of odorants with OBPs as ligands.

Although olfaction cases strong sensations to organisms, the current chemical ex vivo detection methods are yet unable to provide such strong signals in simple ways. It is therefore an on-going goal to provide sensors for odorant detection and determination.

EP 1 233 262 A1 relates to a chemical substance sensor with an odorant binding protein, which may be a dimer. Odorants are detected by fluorescence assays.

US 2003/017618 A1 discloses the use of odorant binding proteins in a sensor chip, which uses changes in the refraction index in a condenser for signal generation.

US 6,598,459 B1 provides an artificial olfactory system with "odorant attachment molecules" being immobilized on a porous material in order to increase the surface area. The sensor is based on a piezoelectric crystal, a surface acoustic wave (SAW) device, or a micro-machined resonator.

US 6,844,197 B1 describes a sensor array with the goal to mimic a human nose detection ability. The human nose is not a very sensitive olfactory system. The system of this document is not directed as absolute odorant detection methods but at a comparison of odorant responses of the system. The sensor itself may be selected from a wide range of sensors, including chemire-sistors, a conducting/nonconducting regions sensor, a SAW sensor, a metal oxide gas sensor, a bulk conducting polymer sensor, a Langmuir-Blodgett film sensor.

US 2012/028820 A1 provides a hybrid sensor array composed of two nanotube field effect transistors (NT FETs), each having a companion transistor that addresses gating in the NT FETs. The nanotubes may be carbon nanotubes functionalized with a biomolecule.

CA 2 666 662 C describes the use of chemical libraries or natural product libraries that are contacted to invertebrate odorant binding proteins to identify compounds that bind to invertebrate odorant binding proteins.

US 2010/188110 A1 relates to a sensor having integrated electrodes in a single sensor configuration, which is operated by alternating current (AC) including periodic electrical excitation signals of the respective multiple frequencies with the same amplitude for detecting analytes in fluids.

US 2012/021932 A1 relates to a multiplexed olfactory receptor-based microsurface plasmon polariton detector. The olfactory receptor has to be stabilized by surfactants on the sensor surface. Surfactants may change the binding behaviour and response of the olfactory receptor deviating from natural behaviour.

A large number of OBPs have been expressed in bacterial systems and their ligand-binding properties have been investigated. The most commonly adopted method involves measuring the fluorescence of a probe able to bind the protein and its decrease consequent to displacement of the probe by a ligand. The fluorescent binding assay requires the availability of a fluorescent probe with good affinity for the protein and presents the inconvenience that what is measured is the ability of a ligand to displace the probe from the complex. This fact could produce results biased towards ligands structurally similar to the probe.

Despite several advances in artificial olfactory detection, there is yet no simple system that is able to simulate the olfactory sensation of sensitive organisms, such as rodents or insects, a system that is able to detect analytes in low concentrations, in an absolute manner without comparative second testing.

Summary of the invention

The goal of the present invention is to provide improved sensor devices with high sensitivity, having improved signal detection or enhancing systems suitable to detect odorant molecules resembling the odorant detection capabilities of living organisms (including non-human organisms).

Surprisingly this goal has been achieved by the present invention, which allowed a highly sensitive detection and concentration measurement of odorant molecules. This allowed for the first time the investigation of the odorant ligand binding of substances in humans but especially in non-human animals. The subject matter of the invention has been defined in the claims.

The present invention relates to a biomolecular sensor device comprising at least one graphene layer as semi-conductor material, wherein said graphene layer comprises bound to its surface a ligand binding protein, with the protein modifying an electrical property of said device, such as impedance or transistor gating of the graphene semi-conductor, especially upon ligand binding and/or conformational change and/or a change in the dipole of said protein. The ligand binding protein can be an olfactory ligand binding protein, such as an OBP or an OR but other ligand binding proteins are also contemplated. The graphene layer is a layer comprising one or more graphene molecules, which may be seen as a graphene "flakes". A graphene semiconductor layer usually comprises up to 15 stacked graphene molecules, preferably 1, 2 or up to 3 stacked graphene molecules. The graphene flakes of the graphene layer are preferably continuous, i.e. contain no holes.

There may be a lipid bilayer being positioned on top of said graphene layer, preferably attached or immobilized to said graphene layer, especially via a linker molecule. Such linkers or other linkers may also be used to bind the ligand binding protein to the graphene molecules. The ligand binding protein, especially if not bound to the graphene layer, may alternatively or in combination be embedded in the lipid bilayer.

In a further related aspect, the invention relates to a graphene layer with an attached lipid bilayer, wherein said graphene layer is in (preferably continuous, i.e. without holes) area-contact with one layer of lipids of said lipid bilayer, in particular the hydrophilic side of the lipids of said layer of lipids, especially preferred wherein said layer of lipids is immobilized to the graphene layer with linker molecules or an adhesive. Said lipid bilayer may comprise embedded therein membrane proteins. Said membrane protein can be the ligand binding protein. The graphene layer may be incorporated into a sensor device.

The invention further provides methods of manufacturing said sensor or graphene layer and uses in analytical methods.

Detailed description of the invention

The following detailed description and preferred embodiments apply to all aspects of the invention as described in the summary of the invention and can be combined with each other without restriction, except where explicitly indicated. E.g. the inventive graphene layer can be used in sensor devices. Sensor devices can be used in methods of detection. Descriptions of methods of detection can be read to require the sensor or graphene layer of the invention to be suitable for such methods. Used elements in methods of manufacture can be read on the provided products to have such elements. Especially preferred embodiments and aspects are defined in the claims.

The inventive high sensitivity could be achieved by using a graphene-layer as substrate for odorant ligand binding proteins, which could be manufactured to a highly sensitive sensor device. These effects that have been discovered for odorant ligand binding proteins also apply for all kinds of ligand binding proteins, thus the present invention is not limited to odorant binding, except where explicitly mentioned. In particular, a new graphene layer has been coated with a lipid bilayer for the first time, which allows the investigation of any lipid binding molecules as detectable agent in assay methods, or the incorporation of membrane proteins and assays for the analysis of their function, e.g. but not limited to the use of (preferably odorant) receptors and the binding of ligands to said receptors.

Graphene layers have been known in the art:

Publication GB 2 485 559 A describes a field-effect transistor (FET) with an array of graphene elements associated with a series of gate electrodes. Individual graphene elements, e.g. flakes or sheets, can be addressed individually. The graphene flakes may be modified with a biomolecule, in particular DNA. DNA molecules are large charged molecules. Single DNA strands bind to equally large and charged complementary DNA strands. DNA binding to its complementary strand thus induces a large change in the charge associated with the graphene layer, which may be detected. This document does not lead to the possibility of detecting small, potentially non-charged molecules on graphene layers.

JP 2012-247189 A, abstract, describes a further graphene modified FET with a functional group adsorbing substance on the edge of the graphene crystal.

US 2011/027853 describes thin-film graphitic layers - composed of several graphene layers - on a substrate in a pattern of choice and modified for improved electrode contact.

US 2012/220053 relates to a FET with a uniform layer of reduced graphene oxide encapsulated semiconductor nanoparticles as gate electrode. These nanoparticles can be attached to biomolecules via linkers comprising polyaromatic ring structures.

US 2013/018599 A1 describes a graphene FET, wherein the graphene layer constitutes the gate. It can be decorated with organic molecules for gas detection.

WO 2009/035647 A1 relates to a graphene sensor comprising an aperture, into which DNA can be placed and translocation, which translocation can be detected.

WO 2010/059687 A2 provides a FET with a gate region comprising a three dimensional matrix comprising, among other possibilities, multiple graphitic/graphene layers. The matrix can be modified with biomolecules as target molecule capture molecules.

WO 2010/097518 A1 relates to a FET with a layer of self-assembled proteins on the surface of a semiconductor gate.

WO 2012/150884 A1 provides a further FET with a gate electrode, said gate electrode is arranged at a distance of a chemically sensitive channel which is formed by a graphene layer on a substrate.

WO 2012/005857 A1 describes graphene sheets with one or more apertures with a membrane forming in said apertures. The artificial membrane includes two lipid monolayers, each contacting one face of the graphene, and the two lipid monolayers form a lipid bilayer within an aperture in the graphene. Within the lipid bilayer, interactions of transmembrane proteins can be tested. However, this document failed to provide a lipid bilayer coated onto the graphene layer.

WO 2012/050646 A2 describes a system for determining transmembrane protein-to-ligand binding properties comprising said proteins in micelles attached to a carbon nanotube, which in turn is linked to electrodes. Example membrane proteins are G-coupled receptors.

Despite the knowledge of the individual components of the inventive sensor, the combination of odorant ligand binding proteins immobilized on a graphene layer has not been known before. Unexpectedly, this combination achieved the high sensitivity required to detect and measure odorant molecules (and other ligands) in a satisfactory method. The inventive concepts further reach beyond the field of odorant detection.

The inventive "ligand binding protein" can be any protein which binds at one time a further agent, referred to as a "ligand". The ligand is a substance that forms a complex with the protein, usually in nature to serve a biological purpose. The ligand can be of any size, it may be another protein, peptide or single amino acid; it may be a nucleic acid, an ion or an organic small molecule. Binding may be a strong binding attraction, leading to the immobilization of the ligand in a binding site of the protein or it may be transient, such as the passage of an ion with interactions with the protein, such as in case of an ion channel or ion transporter. The protein may be an enzyme, which binds to its substrate or cofactor, a receptor, or a ligand binding protein. The protein is preferably a non-structural protein, but in other cases it may be a structural protein. Binding to a structural protein may e.g. be with a further structural protein as ligand. If required for protein stability, the ligand binding protein can be provided in a stabilized environment, such as an environment with a sufficient ionic strength and/or the presence of detergents or, especially in case of membrane proteins, in a lipid bilayer.

Although any size is possible, smaller ligand binding proteins with an increased response (e.g. dipole shift upon ligand binding) are preferred. Such proteins may have a size of at most 150 kDa, at most 100 kDa, at most 90 kDa, at most 80 kDa, at most 70 kDa, at most 60 kDa, at most 50 kDa, at most 40 kDa, at most 30 kDa, or at most 20 kDa. The ligand may be selected from molecules with similar sizes or even smaller molecules with at most 15 kDa, at most 10 kDa, at most 5 kDa, at most 1 kDa, at most 500 Da.

The ligand binding protein may be a membrane protein or a soluble protein. "Soluble protein" is used as accepted terminus for non-membrane proteins. Example membrane proteins are selected from G-coupled proteins, such as olfactory receptors or other G-coupled receptors, integrins, ion channels, transporter proteins, membrane receptors, membrane-associated proteins, redox-proteins. Example soluble proteins are olfactory binding proteins.

The protein, especially in case of a membrane protein, may be placed within a lipid bilayer, which may be placed in contact with the graphene layer. "Within a lipid bilayer", "embedded in a lipid bilayer" or "inserted" therein means that the protein has a domain in contact with the hydrophobic interior of the lipid bilayer, such as a membrane or transmembrane domain. The protein may have further domains outside the lipid bilayer. Artificial lipid bilayers have been proven to be excellent model systems for the investigation of reactions closely associated with the cell surface like ligand-receptor interactions, enzymatic reactions, viral attack and various transport processes. These artificial bilayers have been built by two commonly known systems like black lipid membrane and solid supported lipid bilayers (SLBs). SLBs have been the predominant system for building phospholipid bilayers structures and studying membrane associated processes. It has now been found that it is also possible to provide SLBs on a graphene layer, despite the pi-electron layer associated with graphene, which can repulse the hydrophilic heads of lipids, especially when in concentrated form as found in lipid bilayers.

The sensor is provided in preferred embodiments with a lipid bilayer being positioned on top of said graphene layer, preferably attached or immobilized to the graphene layer, especially via a linker molecule or via a membrane protein. Such linker molecules provide attachment to the lipid bilayer, e.g. by having a lipid of hydrophobic functionality in said linker. Protein binding linkers have a protein binding functionality, which is bound to the protein. Prior art linkers to proteins may be used for the present invention. A linker may e.g. bind to the protein via a covalent bond, such as a Schiff base (azomethine bond). The Schiff base may be reduced to improve stability. A further protein binding functionality are e.g. complex or ionic bonds (e.g. his-tag). A linker binding the graphene layer may have a graphene adhesive functionality. Graphene adhesive functionalities are e.g. aromatic groups, in particular polyaromatic groups, such as a pyrene group. The following linkers are especially contemplated: graphene-lipid, graphene-protein (for both membrane and soluble proteins), lipid-protein.

A special aspect of the present invention is a graphene layer with an attached lipid bilayer. This graphene layer can be provided for a sensor according to the invention, or for different purposes. The inventive graphene layer can be in continuous area contact with one layer of lipids of said lipid bilayer, in particular the hydrophilic side of the lipids of said layer of lipids, especially preferred wherein said layer of lipids is immobilized to the graphene layer with linker molecules. According to any aspect of the invention, the lipid bilayer may comprise inserted therein at least one membrane protein, preferably wherein said membrane protein is a ligand binding protein, especially preferred an olfactory ligand binding protein. Of course, the invention also relates to a biomolecular sensor device comprising a graphene layer with said lipid bilayer, e.g. a sensor as with the ligand binding protein. The lipid bilayer may also comprise a membrane protein therein.

The graphene layer, be it in a sensor or independent therefrom, may be provided on a substrate, including dipolar substrates, insulator, semi-conductors or conductors. Preferred are insulators for uses of the graphene as semi-conductor, such as in a transistor. An example substrate is $SiO_2$. Other uses may comprise the graphene on conductors, e.g. for impedance measurements, which may be modified by ligand binding to the lipid bilayer or ligand binding protein.

The graphene layer is a suitable element for various detection methods. A preferred detection method is electrical detection, e.g. the detection of the electrical resistance, surface plasmon resonance, cyclic voltammetry, impedance or conductivity of a field effect transistor. In such a transistor, the graphene layer preferably is the gate material, wherein it acts as a superconductor between the source and drain electrode to which it may be directly (e.g. attached) or indirectly connected. Also possible is quartz crystal microbalance. All of these methods can be used in the inventive uses and methods for determining a ligand or generally any binding agent to the graphene layer, lipid bilayer or protein.

Thus, in a preferred embodiment of any aspect of the invention, the sensor is being a field-effect transistor comprising a source, a gate and a drain, wherein the gate comprises as semi-conductor the at least one graphene layer. The graphene layer may comprise the ligand binding protein, wherein ligand binding and/or a conformational change and/or dipole change in said protein leads to a modified gating activity of said gate.

Graphene is a suitable gate material for the inventive sensors. The large field effect in graphene enables large shifts in the Fermi level by applying a gate voltage, thus tuning the electronic properties for sensor applications.

The graphene gate may be under the influence of a gate electrode, which may be at a close distance to the graphene layer, such as behind a substrate layer (back gating) or in contact with a liquid, which is in contact with the graphene layer (liquid gating). The gate electrode may or may not be in direct contact with the graphene layer.

The graphene element has attached thereto or associated therewith a protein, and means are provided to permit monitoring of the conductivity of the graphene element, by monitoring, for example, the electrical resistance or conductivity between the associated source and drain electrodes when a potential is applied to the gate, influenced by the protein-ligand binding or interaction. Electrical disturbances induced by the binding of these proteins to the surface of the graphene layer can be measured. These effects are modified by binding or interaction of the ligand with the protein.

In use, the conductivity of the graphene layer may be measured by applying a potential to the gate electrode associated with that element and by measuring the electrical resistance between the source and drain electrodes. The pair of source and drain electrodes are usually substantially non-conductive as a result of the absence of an applied potential to the gate. The graphene as gate on the FET may contact the source and drain electrodes.

In preferred embodiments of the invention, the graphene layer is a continuous sheet of graphene and/or non-porous. Likewise, the lipid bilayer placed on said graphene may be continuous, without any holes or pores, especially over the area in which it is in contact with the graphene layer or single molecular graphene flake of said graphene layer. The present invention thus provides a large area of a lipid bilayer over a graphene layer, which acts over its entire surface as sensor sensitive area, leading to the high sensitivities observed for the invention.

The gate electrode may be a back gate, e.g. as described in US 2013/0018599 A, or a liquid gate, e.g. as described in Misra et al., Applied Physics Letters 90, 052905 (2007). A non-leak Ag/AgCl reference electrode may be used as an electrolyte gate.

The graphene layer according to any aspect of the invention may comprise graphene flakes of about 500 $\mu m^2$ or more, and/or comprises grapheme sheets with an average size of between 200 $\mu m^2$ to 2000 $\mu m^2$, preferably 300 $\mu m^2$ to 1500 $\mu m^2$. "Graphene flake" is used herein to represent single graphene molecules, which form an essentially flat sheet. Sheet formation of the graphene flake can be supported by placement on the substrate. Preferably the graphene layer comprises a thickness of 1, 2, 3, 4, 5, 6, 7, 8, 9 or 10 graphene flakes or sheets, i.e. molecular layers, preferably 1 to 3 graphene flakes or sheets.

The graphene layer, or the area covered by the graphene flakes, preferably covers 30% to 100%, preferably 50% to 95%, especially preferred 70% to 92%, even more preferred 80% to 90%, or the area the sensor area of the substrate. The sensor area of the substrate is the area that can generate a detectible signal when functionalized with the graphene sensor layer. In a FET sensor it is the area between the source and drain electrodes. In a FET, it is especially preferred that the distance between the source and drain electrodes is in the range of 1 $\mu m$ to 100 $\mu m$, especially preferred 3 $\mu m$ to 80 $\mu m$, even more preferred 8 $\mu m$ to 60 $\mu m$, especially preferred 10 $\mu m$ to 40 $\mu m$. It is further preferred that the breadth of the source and/or drain electrodes is larger than the distance of the source and drain electrodes, especially the breadth of the electrodes may be at least 100 $\mu m$, at least 150 $\mu m$, at least 200 $\mu m$, at least 300 $\mu m$, at least 500 $\mu m$, such as in the range of 120 $\mu m$ to 2000 $\mu m$.

As said, a ligand binding protein may be placed onto said graphene layer. Said protein can be a membrane or protein or not a membrane protein. Non-membrane proteins may be bound to a membrane via a linker (e.g. the lipid-protein linker mentioned above). The protein can be placed onto the graphene layer either directly, via a linker or within a lipid layer, including a lipid monolayer but especially a lipid bilayer that is placed on said graphene layer.

In preferred embodiments of the invention said protein is an odorant binding protein (OBP) or an olfactory receptor (OR). Thanks to genome sequencing, a very large number of OBP sequences are available and three-dimensional structures have been solved for 20 of them (e.g. Wogulis et al. Biochemical and Biophysical Research Communications 339(1), 157-164 2006). They usually have a common folding of six alpha-helical domains, further stabilized by three interlocked disulfide bridges. Another important characteristic of OBPs is its extreme stability to temperature, solvents and proteolysis making such proteins ideal tools for biosensors to be used in environmental monitoring. The same applies to olfactory receptors. A thousand OR sequences are known per organism (Gaillard et al., CMLS, Cell. Mol. Life Sci. 61 (2004) 456 - 469). They are encoded by the largest multigene family (~1000 members) in the genome of mammals and belong to the heptahelical G-protein-coupled receptor (GPCR) super-family.

A ligand to an olfactory ligand binding protein is considered herein as an "odorant".

Odorants may be selected from a wide range of substances, e.g. explosives in bomb detection, biological repellents and attractants, especially insect repellents and insect attractants, environmental toxins, biomedicals, cosmetics and/or perfumes, pharmaceuticals, combustible gas, combustion emissions, smoke, beverage contents, food contents, and agricultural products, such as for freshness detection, fermentation products, and flavors, drugs and other illegal substances, diesel/gasoline/aviation fuel, odorants in body fluids, infectious disease emissions, breath components, fragrances.

The protein can be placed, but is preferably immobilized, onto the graphene layer by a linker molecule, preferably a linker molecule comprising a graphene binding moiety, such as an aromatic moiety, e.g. a pyrene group. The graphene binding moiety preferably binds to graphene by pi-pi electron stacking.

Preferred proteins can be selected from vertebrate and non-vertebrate proteins, preferably insect proteins, especially insect OBPs. In general, the protein, e.g. an OBP or OR, may be from a mammal, especially a rodent or domestic animal such as a dog, cat, cow or horse, reptile, amphibian, marsupial, fish or insect.

The present invention also refers to a use of a sensor or graphene layer according to the invention in a sensor and/or for detecting an ligand of the ligand binding protein or generally any substance that attaches to the lipid bilayer (with and without membrane proteins) or the ligands binding proteins. Preferably, ligand binding changes the electric dipole moment of the protein-ligand complex as compared to the protein without the ligand. Said binding reaction can cause a change in an electrical property of the graphene layer which is detected.

Associated with this use, the invention also provides a method of determining the interaction of an analyte ligand with a protein using a sensor according to any aspect of the invention, with said protein being bound to the surface of the graphene layer of the sensor, wherein said analyte potentially interacts with said protein, whereby an electrical property of said device is modified and said modification is determined. The electrical property can e.g. be an impedance or transistor gating of the graphene semi-conductor. In particular preferred, said electrical property is electric resistance or conductivity between a source and drain electrode of a field effect transistor, when a potential is applied to a gate electrode, wherein said potential is modified by said interaction of the analyte with the protein. Especially, the analyte may be the ligand of the ligand binding protein. In case of a FET, the source-drain voltage may be any suitable voltage that allows conductance when applying a gate voltage and no or minor conductance without a gate voltage. Example source-drain voltages are in the range of 1 mV to 1 V, especially preferred in the range 10 mV to 500 mV, even more preferred in a range of 20 mV to 100 mV, such as about 50 mV.

In said method, the potential analyte/ligand is contacted with said sensor, especially the modified graphene layer, in sufficient time and under conditions to allow binding of said analyte/ligand. This interaction can be by transferring a sample potentially comprising the analyte/ligand to the modified graphene layer. This can be performed non-continuously, e.g. in batch, or continuously, e.g. in a flow-cell. Preferably the sample is continuously transferred through a detection cell containing the inventive sensor at a flow rate of 10 $\mu$L/min to 2000 $\mu$L/min, preferably of 100 to 800 $\mu$L/min, more preferred of about 300 $\mu$L/min.

In special embodiments the sample is a fluid, especially a liquid, preferably an aqueous liquid.

In particular preferred embodiments the sample is measured at different concentration, e.g. dilutions. By diluting the sample it is possible to easily determine different signals intensified, which can be used to determine analyte/ligand concentrations in the (undiluted) sample.

Also provided is a method for the manufacture of a graphene layer according to the invention (with/without membrane proteins) comprising the step of providing a sheet of graphene on a substrate, optionally wherein said graphene is attached to said substrate with an adhesive, preferably an aminosilane such as APTES, applying a lipid linker, with a graphene adhesive functionality to said graphene layer, and applying lipid molecules to said graphene, preferably in form of lipid vesicles. The substrate can be a non-conductor, a semiconductor or a conductor, preferably a non-conductor for later FET uses.

Also provided is a method of manufacture of a sensor according to the invention comprising providing the step of providing a sheet of graphene on a substrate, optionally wherein said graphene is attached to said substrate with an adhesive, preferably an aminosilane such as APTES, said substrate is a non-conductor, a semiconductor or a conductor, preferably a non-conductor for later FET uses, attaching a protein to said graphene layer. Essentially two methods to attach the protein are contemplated: either i) further attaching a lipid bilayer on said graphene layer, introducing a membrane protein into said lipid bilayer, or ii) binding a linker with a graphene adhesive functionality on said graphene layer, and attaching a protein to said graphene bound linker. Graphene adhesive functionalities are e.g. aromatic groups, in particular polyaromatic groups, such as a pyrene group. These can be provided on linkers, which bind to graphene according to any aspect of the invention, including in case of graphene-protein linkers or graphene lipid linkers. Graphene-lipid linkers are modified lipids that can be used to stabilize a lipid bilayer on the graphene layer. Preferably both, protein-graphene linkers and graphene-lipid linkers, are used in

a sensor or graphene layer. The protein can be bound to the linker by well-known functional groups, e.g. which form a covalent bond (e.g. Schiff base) to the protein or a complex or ionic bond (e.g. his-tag). It is also possible to generate a membrane on the graphene layer after protein binding. In this case, especially when the protein is a membrane protein, the protein may function as an anchor on which the lipid molecules assemble to form the lipid bilayer. In other aspects of the invention, the graphene layer is not modified by a lipid layer, especially in case of soluble olfactory binding proteins.

The present invention is further illustrated by the following examples and figures, without being limited to these embodiments of the invention. Of course each single element of these further embodiments can be combined with each aspect of the invention as described above.

**Figures:**

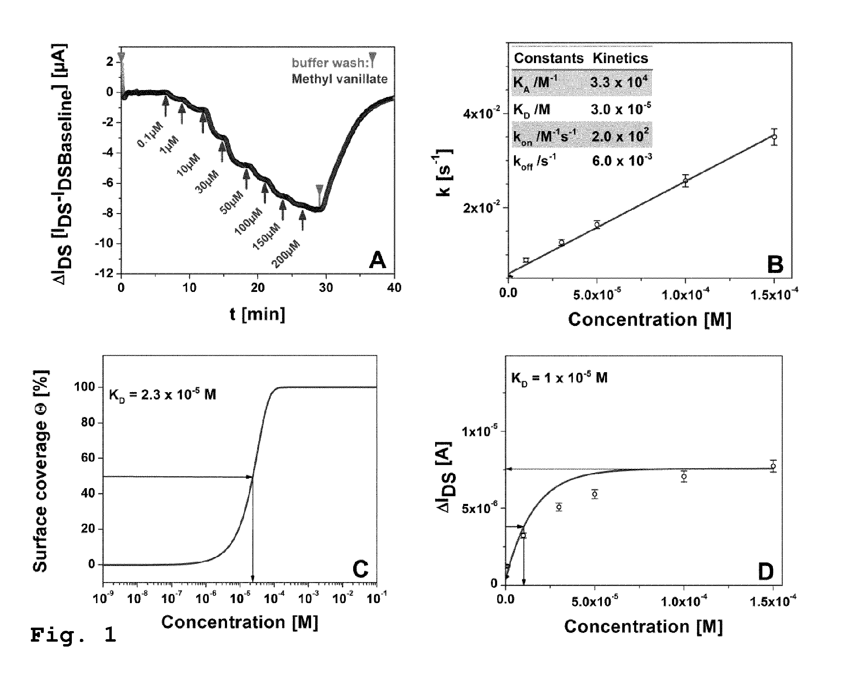

**Fig. 1.** (A) Titration curve for methyl vallinate/OBP14 interaction using rGO-based sensor. The association and dissociation rate constants were derived from the fit to the data points (red solid curve) and are given in Table 2. Blue arrows indicate a buffer wash step. (B) $k = k_{on}[Od] + k_{off}$ obtained from the fitted data of (A) as a function of target concentration. (C) Langmuir isotherm plotted as surface coverage vs. odorant concentration for the data points taken from (A). (D) Plot of the maximum current drop expressed as $\Delta I_{DS}$ taken from (A) vs. target odorant concentration [$Od$].

**Fig. 2.** Titration of immobilised OBP14 with eugenol (A), citral (B) and sulcatone (C). Green arrows indicate the addition of ligand solutions. Blue arrow indicates buffer wash. The association and dissociation constants were derived from red solid curves and are reported in Table 1. The Langmuir isotherm plots (D) of eugenol (solid), citral (dashed) and sulcatone (dotted) show significantly different $C_{1/2}$ ($K_D$) values (surface coverage $\Theta$ = 50%).

**Fig. 3.** (A-C) Real-time response curves of OBP14-functionalized biosensor to ligands possessing phenolic basic structure at different concentrations. Methyl eugenol, which does not present a free hydroxyl group, is a much weaker ligand (D). Red arrows indicate the additions of ligand solutions. Blue arrows indicate buffer wash. The association and dissociation rate constants were derived from the fits to the data points (red solid curves) and are given in Table 2.

**Fig. 4.** Consistency check of the detection system. Three different concentration of eugenol solution were introduced to the detection surface, yielding in concentration independent $k_{off}$ values as predicted by the Langmuir model.

**Fig 5.** Membranes on graphene (reduced graphene oxide)

**Fig 6.** a) SPR Kinetic trace hyb-tBLM; b) Bode plots hyb-tBLM. (Insert - Circuit diagram); c) QCM-D hyb-tBLM. 1) PBS stabilization, 2) addition of surfactant, 3) addition of Pyrene lipids, 4) PBS buffer wash. 5) ex-situ dialysis with DPhyPC vesicles and bio beads; d) Thickness change ($\Delta$t) fit hyb-tBLM.

**Fig. 7.** a) SPR Kinetic trace hyb-ptBLM; b) Bode plots hyb-ptBLM. (Insert - circuit diagram); c) QCM-D plot hyb-ptBLM. 1) PTNTA layer stabilization, 2) addition of surfactant, 3) addition of CcO, 4) surfactant wash. 5) ex-situ dialysis with lipids in surfactant and bio beads, 6) buffer wash; d) Thickness change ($\Delta$t) fit hyb-ptBLM.

**Fig. 8.** Angle scan highlighting hyb-tBLM formation with optical thickness of the layers derived from fit shown in the insert. (Fit calculated with refractive index values of prism: 1.845, gold: 0.17, graphene: 2.1, surfactant: 1.334, pyrene lipids: 1,434, membrane: 1,434 and kappa values of gold: 3.61, graphene: 0.56.)

**Fig. 9.** Dynamic light scattering measurement for two samples of DPhyPC small unilamellar vesicles (SUVs) showing 100nm diameter.

**Fig. 10.** Nyquist Plot highlighting change in real impedance (Z') vs imaginary impedance (-Z") for hyb-tBLM system (shown by colored squares). Insert shows the circuit diagram used to model the fits for individual layers (shown as colored lines in legend).

**Fig. 11.** QCM-D highlighting Pyrene lipids layer formation for hyb-tBLM: 1) PBS buffer stabilization, 2) addition of surfactant, 3) addition of Pyrene lipids, 4) PBS buffer wash forming stable PLL.

**Fig. 12.** Angle scan highlighting hyb-ptBLM formation with optical thickness of the layers derived from fit shown in the insert. (Fit calculated with refractive index values of prism: 1.845, chromium: 3.13 gold: 0.17, graphene: 2.1, surfactant: 1.334, CcO: 1.434, membrane: 1.434 and kappa values of chromium: 3.52 gold: 3.61, graphene: 0.56).

**Fig. 13.** Nyquist Plot highlighting change in real impedance (Z') vs imaginary impedance (-Z") for hyb-ptBLM system (shown by colored squares). Insert shows the circuit diagram used to model the fits for individual layers (shown as colored lines in legend).

**Fig. 14.** Cyclic Voltammogram of redox protein CcO within the hyb-ptBLM system.

**Fig. 15.** hyb-ptBLM QCM-D 1) PBS buffer stabilization, 2) addition of Surfactant, 3) addition of CcO, 4) Surfactant wash.

## Examples:

All reagents were purchased from Sigma-Aldrich unless otherwise indicated and used without further purifications.

### Example 1: Cloning and purification of OBP14

Odorant binding protein (OBP14) was expressed in a bacterial system using established protocols (Dani et al. J Proteome Res 9(4), 2010: 1822-1833; Iovinella et al. Journal of Proteome Research 10(8), 2011: 3439-3449). Purification of the protein was accomplished using a combination of conventional chromatographic techniques followed by a final gel filtration step on Superose-12 (GE-Healthcare) as previously described in standard protocols. The purity of the protein was checked by SDS-PAGE and the size of ~14.0 kDa was determined for OBP14 (Iovinella et al., supra).

### Example 2. Preparation of odorant solutions

Odorants were prepared as 5 mM stock solutions in phosphate buffer (1mM PBS, pH = 8.0) with an addition of 0.01% ethanol (purity $\geq$ 99.9%) and further diluted (from $10^{-3}$ M to $10^{-6}$ M) by successive dilutions in PBS buffer. All solutions were freshly prepared on the day of the experiment.

A broad spectrum of ligands was tested known as typical pollen odorants like the benzenoids Eugenol (4-Allyl-2-methoxyphenol) and Methyl eugenol (1,2-Dimethoxy-4-prop-2-enylbenzene) or Sulcatone (6-Methylhept-5-en-2-one), an irregular terpene which was also identified in propolis. The binding profile of another propolis compound, Coniferyl aldehyde, ((E)-3-(4-hydroxy-3-methoxyphenyl) prop-2-enal) was investigated as well. Moreover, a few floral volatiles, known to be attractive for bees, were tested. These include Vanillin (4-Hydroxy-3-methoxybenzaldehyd), Vanillyl acetone (4-(4-hydroxy-3-methoxyphenyl)butan-2-one), Citral ((2E)-3,7-dimethylocta-2,6-dienal) and Geraniol ((2E)-3,7-Dimethylocta-2,6-dien-1-ol), the latter two were also identified as Nasonov pheromone compounds. In addition, two honey flavours, namely Homovanillic acid (2-(4-Hydroxy-3-methoxy-phenyl)acetic acid) and Methyl vanillate (Methyl 4-hydroxy-3-methoxybenzoate) as well as Isoamyl acetate (3-methylbutyl acetate), an alarm pheromone, were also tested.

### Example 3. Fabrication of OBP based biosensor devices

Reduced graphene oxide field-effect transistor (rGO-FET) devices were fabricated according to (Larisika et al. Materials Chemistry and Physics 136(2-3), 2012: 304-308) with a modified channel width of 30 $\mu$m. For the detection of different odorants, the rGO-based biosensors were initially functionalized with OBP14. For the attachment of the protein to the sensing area, the graphene surface was chemically modified by a bi-functional linker, 1-Pyrenebutanoic acid succinimidyl ester (PBSE). On one end the linker firmly attaches to the graphene surface through n-n interactions with a pyrene group and on the other hand covalently reacts with the amino group of the protein to form an amide bond. Therefore, 20 $\mu$L of a 5 $\mu$M PBSE solution in THF was placed onto the rGO-FET channel. Hence, 10 $\mu$L of a 10 $\mu$M OBP14 solution in PBS buffer (10 mM; pH = 8.0) was deposited onto the detection area and incubated for 2h at 4°C.

### Example 4. Electrical measurements

The evaluation of OBP binding affinities to various odorants was performed under these standardized conditions: Electrical measurements were performed using a Keithley 4200 semiconductor characterization system. An Ag/AgCl reference electrode (Flex ref, World Precision Instruments) was used to operate the FET device in liquid gate configuration with a constant gate bias (Vg) of -0.6 V and a constant source-drain bias ($I_{DS}$) of 0.05 V. A flow rate of 300 $\mu$L/min was chosen to minimize mass transfer limitations of the analytes to the sensor surface. The general procedure of the whole titration experiment started with continuously flushing the detection area with pure buffer (1mM PBS, pH = 8.0), until a stable baseline of drain current was established. Subsequently, the biosensor was titrated with different odorant con-

centrations ($0.1*10^{-6}$ M - $4*10^{-3}$ M) and the change in source-drain current ($\Delta I_{DS}$) was monitored in real time. Here, the lowest odorant concentration was injected first and the odorant was allowed to interact with the surface immobilized OBP14 to reach equilibrium, as indicated by a constant drain current. This process was repeated with odorant solutions of higher concentrations, until the surface was fully saturated with the target analyte, demonstrated as no significant change in drain current ($I_{DS}$). Surface titration experiments were terminated by a final washing step using pure buffer in order to dissociate the formed OBP14-Odorant complex, resulting in almost complete restoration of the initial baseline current. As observed in almost all biosensor devices, a slight drift in source-drain current was observed with time. For this reason, the drain current response curves were normalized by subtraction to the baseline current. Kinetic parameters of the above described titration experiments were analysed using the Langmuir model.

## Example 5. Results and discussion

When the rGO-FETs functionalized with OBP14 were exposed to different odorants, we measured changes in the source-drain current ($\Delta I_{DS}$) (conductance) of the rGO-FETs. This suggests that binding of the ligand inside the core of the protein affects the electrical charge distribution of OBP14 immobilized on p-type semiconductor. To evaluate the amount of odorants selectively adsorbed on the OBP-covered surface surface titration experiments were performed. Here, the sensor response was measured by monitoring changes in source-drain current ($\Delta I_{DS}$) with time, when different concentrations of specific odorant solutions were introduced to the sensing area. The electrical response of OBP-coated FET devices to various odorant solutions can be analyzed using the Langmuir isotherm model.

## Example 6. Langmuir isotherm theory

For the evaluation of binding kinetics we assume that the reaction on the surface of the biosensor follows the Langmuir adsorption model (Langmuir, Journal of the American Chemical Society 40(9), 1918: 1361-1403) with a dynamic equilibrium

$$Od \;+\; OBP14 \;\; \underset{k_{off}}{\overset{k_{on}}{\rightleftharpoons}} \;\; Od\text{-}OBP14$$

for the formation of a complex *Od-OBP14* between immobilized protein (*OBP14*) and odorant (*Od*). In Fig. 1 titration experiments and data analysis are shown for an exemplified odorant, namely methyl vanillate. To ensure reliable determination of binding affinities by biosensor devices, kinetic data for all odorants were analysed and tested for plausibility.

For the titration experiment (Fig. 1A), the changes in drain current ($\Delta I_{DS}$), measured as a function of time during formation of the complex Od-OBP14, can be utilized to determine the association ($k_{on}$) and dissociation ($k_{off}$) rate constants using equation (1) and (2), based on the Langmuir assumption,

$$I_{Od}(t) = (I_{Od,max} - I_0) * ( 1 - \exp ((- k_{on} * Od + k_{off}) * t)) \qquad \text{equation (1)}$$

where $I_{Od,max}$ is the equilibrium value of $I_{DS}$ for each given odorant concentration [*Od*], and $I_0$ is the baseline current. The association kinetics is quantified with respect to the relative [*Od*]. All association data points of the titration curve were fitted with equation (1) in the form: $I_{Od}(t) = (I_{Od,max} - I_0) * ( 1 - \exp (-k * t))$ to obtain $k_{on}$ values for each odorant concentration, where the reaction rate constant $k$ is defined as $k = k_{on} * [Od] + k_{off}$. The final dissociation experiment (at [*OD*] = 0 μM) is described by equation (2) as follows:

$$I_{Od}(t) = (I_{Od,max} - I_0) * \exp (- k_{off} * t) \qquad \text{equation (2)}$$

All dissociation data points of the real-time measurement were exponentially fitted with equation (2) (as indicated with a red solid line in Fig. 1A) to obtain a single $k_{off}$ rate constant. Affinity constant ($K_A$) and dissociation constant ($K_D$) can be calculated using the relation $K_A = k_{on}/k_{off}$ and $K_D = 1/K_A$. From the kinetic measurement a $K_D$ value of $22.0 \pm 4.5 *10^{-6}$ M for methyl vanillate was obtained. All kinetic constants are reported in Table 2.

A linear relationship between reaction rate constant $k$ and odorant concentrations, as shown in Fig. 1B is a good indication that kinetic data obtained by titration experiments are reliable. From this linear relationship a $K_D$ of $3.0*10^{-5}$ M was determined. Binding affinities obtained by the different approaches are in good agreement for the odorant methyl vanillate as well as for all other tested ligands (cf. Table 1+2).

Further support for the experimental data (and for the underlying model) comes from the plot of surface coverage *SC (e)* versus odorant concentration displayed in Fig. 1C. The surface coverage was calculated and fitted using equation (3). The half saturation concentration, indicated as 50% SC, yielded a $K_D$ value of 2.4 *$10^{-5}$ M for the tested ligand, which is consistent with that determined from the titration curve (see Table 2).

$$SC\ (\theta) = (K_A * [Od]) / (1 + K_A * [Od])$$ equation (3)

Fig. 1D presents the Langmuir isotherm of the change in drain current $\Delta I_{DS}$ as a function of odorant concentration [Od]. This approach produced a $K_D = 1.0 * 10^{-5}$ M, in good agreement with the above calculation.

**Example 7. Binding profiles of odorants to OBP**

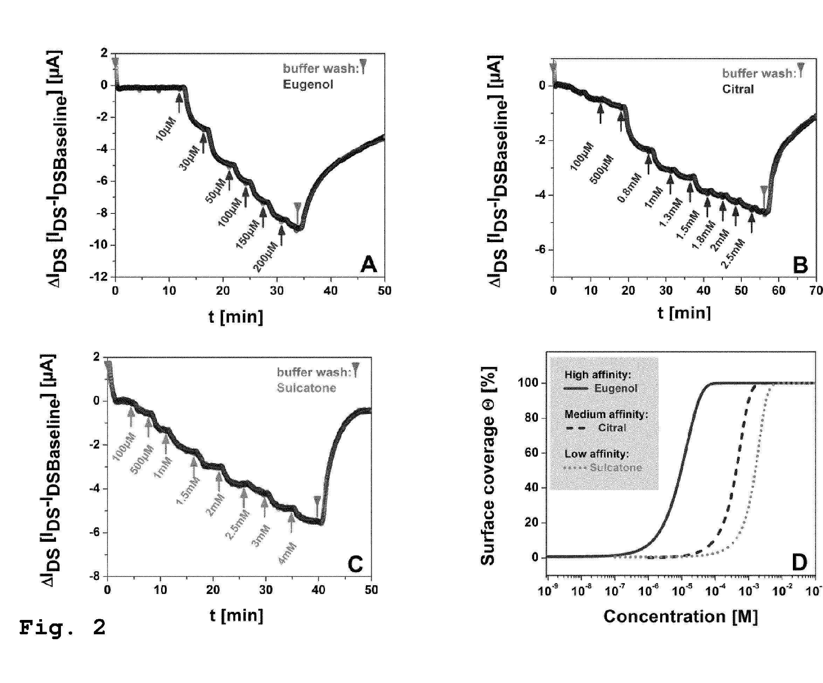

Fig. 2 represents the real-time sensor responses to three different ligands. The results clearly demonstrate the capability of the biosensor to discriminate between good, moderate and poor ligands through their binding profiles to the OBP-based biosensors.

By a step-wise increase of the target odorant concentration [Od] a corresponding decrease in the $I_{DS}$ was observed with a relative drain current plateau-value being characteristic for the corresponding bulk concentration. The $I_{DS}$ was measured as a function of time until the equilibrium between the bulk concentration and the corresponding surface coverage was reached. Hence, the saturation state of the sensor was achieved at a rather high concentration for citral and sulcatone compared to eugenol. The Langmuir isotherms describing the three different binding profiles (Fig. 2A-C) are displayed in Fig. 2D by the three different S-shaped binding curves (solid: eugenol, dashed: citral, dotted: sulcatone). They are separated from each other by 1-3 orders of magnitude in their respective affinity constants ($K_A$), as evaluated from the half-saturation values (surface coverage $\Theta$=50%). Kinetic data obtained by the titration experiments fitted (red curves) the Langmuir model described above with $K_D$ of 9.2 *$10^{-6}$ M for eugenol, 5.9 *$10^{-4}$ M for citral and 1.4 *$10^{-3}$M for sulcatone (cf. Table 1).

**Table 1:** Rate constants of various odorants determined from the titration measurements (Fig. 1 and Fig. 2 and S5-S6) using the Langmuir model.

| | Homovanillic acid | Eugenol | Citral | Isoamyl acetate | Geraniol | Sulcatone |
|---|---|---|---|---|---|---|
| $10^2$ [$K_A$] /$M^{-1}$ | 1540 ± 140 | 1087 ± 91 | 16.97 ± 0.02 | 12.33 ± 0.86 | 8.96 ± 0.04 | 7.07 ± 0.11 |
| $10^{-6}$[$K_D$] /M | 6.5 ± 0.7 | 9.2 ± 0.7 | 589.2 ± 0.6 | 814 ± 58 | 1120 ± 5 | 1410 ± 21 |
| [$k_{on}$]/$M^1 s^{-1}$ | 1206 ± 110 | 326 ± 27 | 5.96 ± 0.006 | 8.3 ± 0.6 | 6.89 ± 0.03 | 5.23 ± 0.08 |
| [$k_{off}$] /$s^{-1}$ | 0.0078 | 0.0030 | 0.0035 | 0.0068 | 0.0077 | 0.0074 |

Further support for these kinetic data (and for the assumed underlying model) comes from the Langmuir adsorption isotherm plots which resulted in $K_D$ values of 1.9*$10^{-6}$ M for eugenol, 5.5*$10^{-4}$ M for citral and 1.5*$10^{-3}$ M for sulcatone showing an excellent agreement with the titration results.

Surface titration with other ligands, namely homovanillic acid, isoamyl acetate and geraniol were performed in a similar manner.

As it is acknowledged, honeybees, as other social insects, recognize and react to a wide range of different odorants and pheromone molecules mediating communication in honeybee hives or locating floral rewards (pollen, nectar, ect.). Here, OBPs work as a first filter for odor recognition. According to our results we could show that the fabricated biosensor refers to a possible functional role of OBP14 in odor recognition by detecting various odorants with dissociation constants $K_D$ in the micromolar range.

Comparing the different binding properties of OBP14 towards the chosen ligands (cf. Table 1) our results confirm the general tendency previously demonstrated by fluorescence measurements that eugenol exhibits high affinity to OBP14, whereas citral and geraniol are moderate or weak binders. However, for two compounds, namely homovanillic acid (Table 1) and coniferyl aldehyde (Table 2), affinity constants to OBP14 could only be determined by our label-free method, since both ligands did not show good affinity to OBP14 according to fluorescent displacement assays (Iovinella et al., supra).

Considering the structure of OBP14 in complex with a strong ligand, eugenol, it has been shown that an ensemble of hydrogen bonds including a strong hydrogen bond between the hydroxyl group of the ligand and Lys111 C=O moiety of the protein binding site are responsible for the favorable binding of this ligand. However, a recognizable pattern in terms of a preferential class of odorants to OBP14 could not be detected so far, although structurally similar ligands to eugenol, including homovanillic acid and coniferyl aldehyde have been tested (Iovinella et al., supra).

Our measurement results indicate that cyclic bulky compounds like eugenol and homovanillic acid displaying a hydrogen bond acceptor seem to be favoured for OBP14, whereas elongated compounds, such as isoamyl acetate or sulcatone behave poorly.

**Example 8. Binding spectra and determination of affinity constants for ligands with phenolic basic structure**

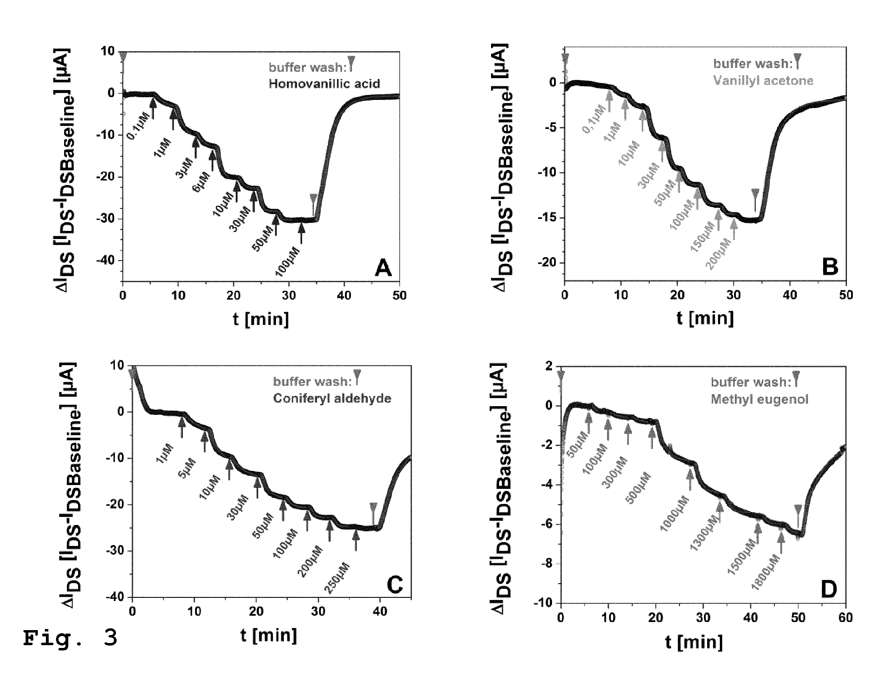

To check, whether OBP14 preferentially binds ligands with specific structural properties, other odorants possessing phenolic structure were tested. As a control, methyl eugenol was also included. Comparing the titration curves of three ligands with phenolic based structure (Fig. 3A-C) to OBP14 with the one of methyl eugenol, an odorant lacking a hydroxyl residue (Fig. 3D), we found that with methyl eugenol equilibrium was achieved at a concentration about 10-fold higher with respect to the other odorants.

Rate constants for all odorants with phenolic chemical structure, except homovanillic acid (cf. Table 1), as well as these for methyl eugenol calculated from the titration experiments (Fig. 3) are displayed in Table 2. From the full set of data obtained by our biosensor, we can conclude that OBP14 preferentially binds odorants containing phenolic structures and that the presence of a free hydroxyl group on the molecule plays an important role on the fitting of these ligands to OBP14.

**Table 2:** Rate constants of odorants with similar chemical structure determined from the titration measurements (Fig. 1 and Fig. 2) using the Lanqmuir model fit.

|  | Eugenol | Methyl eugenol | Coniferyl aldehyde | Vanillyl acetone | Methyl vanillate | Vanillin |
|---|---|---|---|---|---|---|
| $10^2$ [$K_A$] /$M^{-1}$ | $1087 \pm 91$ | $19.23 \pm 0.03$ | $1240 \pm 630$ | $409.7 \pm 0.3$ | $479 \pm 136$ | $483 \pm 268$ |
| $10^{-6}$ [$K_D$] /M | $9.2 \pm 0.7$ | $520.0 \pm 0.1$ | $9.1 \pm 2.3$ | $24.4 \pm 0.02$ | $22.0 \pm 4.5$ | $23.8 \pm 6$ |
| [$k_{on}$] / $M^{-1}s^{-1}$ | $326 \pm 27$ | $8.92 \pm 0.01$ | $370 \pm 190$ | $286.8 \pm 0.2$ | $239 \pm 68$ | $191 \pm 111$ |
| [ $k_{off}$] / $s^{-1}$ | 0.0030 | 0.0046 | 0.003 | 0.007 | 0.005 | 0.0038 |

Our presumption is supported by a control experiment. For this purpose, the response signals of eugenol and methyl eugenol to OBP14 were investigated on the same sensor device. As expected, the real-time response ($\Delta I_{DS}$) of the biosensor to 5 $\mu$M eugenol solution was significantly higher than to a 10 fold higher concentrated methyl eugenol solution. This result also shows that our biosensor responded selectively and could distinguish odorants with very similar chemical structure. Between eugenol and methyl eugenol, the dissociation constant of $K_D = 1.9*10^{-5}$ M for eugenol compared to a $K_D$ value of $5.9*10^{-4}$ M for methyl eugenol clearly indicates that a free hydroxyl group of ligand structure has an influence on the interaction with the binding pocket of the protein.

**Example 9. Consistency check of the detection system**

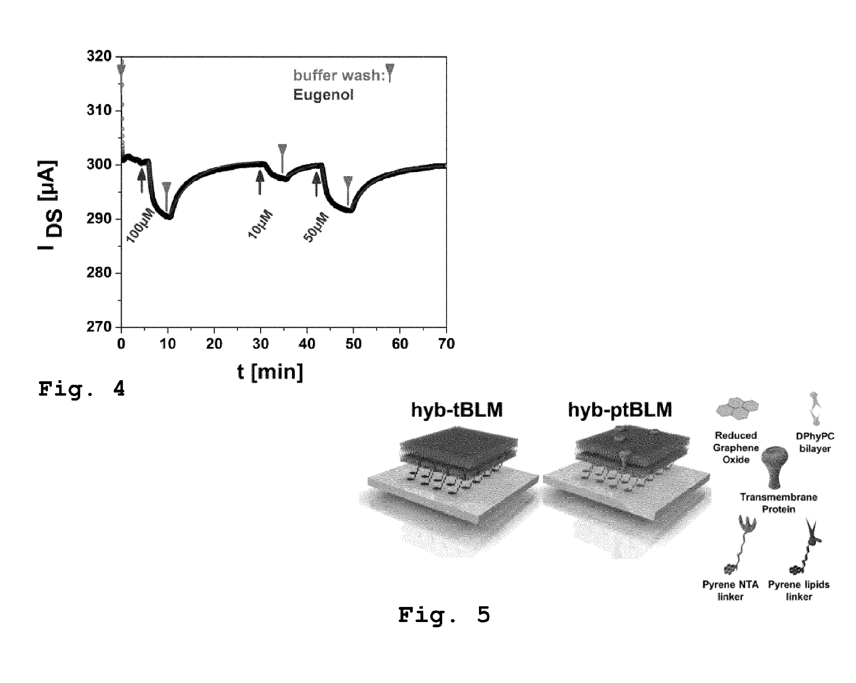

In a control experiment (Fig. 4) association and dissociation phases of a protein-ligand interaction were measured systematically at different concentrations of target odorant solution (eugenol) with regeneration steps between measurements. Since each experiment starts from a bare probe, reliable $k_{on}$ and $k_{off}$ values can be obtained. An analysis of this control measurement revealed that association process, indeed, speeds up linearly with increasing ligand concentration, while dissociation rate constant, $k_{off}$, is concentration (and coverage) independent as predicted by the Langmuir model. In fact, at all concentrations of eugenol we obtained a $k_{off}$ value of 0.003 $s^{-1}$ after nonlinear curve fitting of the dissociation processes.

Besides a successful detection of various floral and pollen specific odorants with a detection limit of micromole, our biosensor could classify the tested ligands as good, moderate and low affinity odorants based on their different affinity constants determined. Dissociation constants ($K_D$) for best ligands were determined in the micromolar range, as known for OBPs. For two compounds, namely homovanillic acid and coniferyl aldehyde, affinity constants could currently be determined by our label-free method, which did not succeed by commonly used fluorescent displacement assays so far. Moreover, the present results provide evidence that phenol-based structured compounds, like homovanillic acid, eugenol or coniferyl aldehyde seem to be favoured by OBP14. Further support for our assumption of preferential odour pattern provides the different binding profiles of eugenol and methyl eugenol, two pollen compounds, which differ only in a free hydroxyl group. The rGO-based biosensor functionalized with OBP responded to a broad but selective spectrum of odorants that has not been detectable so far.

**Example 11. Graphene - lipid bilayer constructs**

Hybrid tethered bilayer lipid membrane (hyb-tBLM) and hybrid protein tethered bilayer lipid membrane (hyb-ptBLM) on reduced graphene oxide have been created (Fig. 5), improving previous artificial membrane systems, with its ease of construction, reproducibility, easily modifiable surface for oriented immobilization of the protein via his-tag technique thus preserving the protein biological function within the artificial bilayer platform.

The hyb-tBLM and hyb-ptBLM platform over reduced graphene oxide (rGO) surface was created via a novel technique of step-wise assembly of each component of the platform, while simultaneously monitoring and establishing the system using three techniques namely surface plasmon resonance (SPR), electro-impedance spectroscopy (EIS) and quartz crystal micro-balance with dissipation (QCM-D).

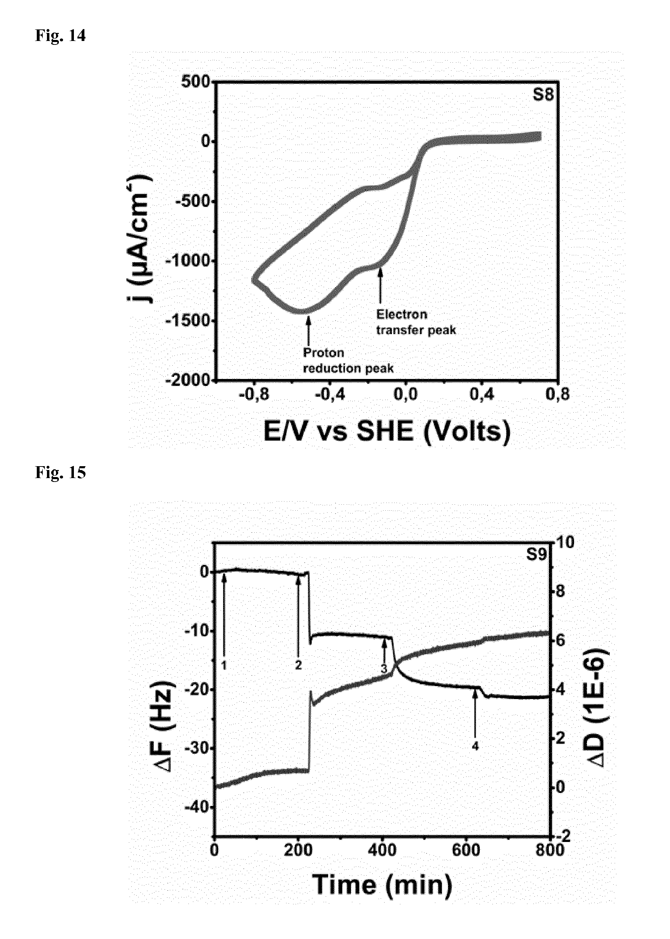

The intention behind construction of hyb-tBLM is to prove the formation of lipid bilayer over the Pyrene lipid layer (PLL) constructed upon the rGO surface, thus engineering a versatile system to study membrane associated interactions for fundamental research or for potential pharmaceutical applications. For our hyb-ptBLM platform, using a redox protein (Cytochrome c oxidase (CcO) from Rhodobacter sphaeroides with his-tag attached to subunit I) as a bench-mark system, we prove that the protein maintains its biological function within our functional lipid bilayer model. This has been demonstrated by the cyclic voltammetry (CV) technique (Fig. 14). The key feature of our rGO membrane sensors is the facile method of its production and the additional advantage of 98% transparency of the rGO which makes it suitable for fabricating transparent electrodes. Thus, measuring the functionality of complex membrane proteins and to create a sensitive, label-free rGO membrane sensor platform which could be read-out by optical and electrochemical methods simultaneously is possible.

In comparison to label free techniques like Surface plasmon resonance (SPR) which need design of special nanoarray systems to embed membrane receptors for studying ligand interaction and Electro-impedance spectroscopy (EIS) which have the main challenge of solving nonspecific response, our membrane architectures also allows for label free analysis and provide functional data covering a broad range of ligand-membrane receptor interactions, coupled with robust, economic and facile integration into Bio-nanoelectronic devices.

**Example 12. Reduced Graphene oxide synthesis and deposition on gold surface**

Gold surface was prepared by evaporation (Auto 306, Hind Hi-Vac) of chromium (3.2 nm) and gold (45-50 nm) onto a cleaned glass slide (BK7 grade, VWR) at 2 to 3 $E^{-7}$ mbar vacuum with evaporation rates of 0.1 Å/sec for both chromium and gold deposition. The gold surface was functionalized with 1% cysteamine solution in ethanol for 1 hour, then rinsed thoroughly with ethanol after and then deposited with exfoliated graphene oxide by filter press method. Polyethersulfone (PES) filters were deposited with graphene oxide solution and then the glass slides were pressed on these filters. This procedure was repeated three times. These slides were treated with hydrazine monohydrate (Sigma Aldrich).

The rGO surface coverage was evaluated using scanning electron microscopy (SEM) method where almost 90% rGO flake coverage was obtained.

**Example 13. Solution preparation for hyb-tBLM and hyb-ptBLM systems**

The buffer was autoclaved at 121°C before preparing the solutions below. All surfactant and lipid solutions are stored at -4°C. All the solutions and buffer were prepared freshly for each experiment. 40mM Nickel chloride solution ($NiCl_2.6H_2O$) 500 mL deionized $H_2O$ was added to 1.5 mL $CH_3COOH$ (Sigma Aldrich) + 4.75 gms $NiCl_2.6H_2O$ (Sigma Aldrich). The solutions were mixed thoroughly and then pH was adjusted to 5.5 with KOH (Sigma Aldrich). PBS Buffer pH 8.0 - 0.05M $K_2HPO_4$ (8.71 gms) and 0.1M KCl (7.455 gms) were mixed together with 1Ltr of deionized water and the pH of solution to was adjusted to 8.0.

Surfactant solution (0.1% DDM solution) - 250 mgs DDM (Sigma-Aldrich) + 250 mL PBS buffer pH 8.0 were mixed together to prepare 0.1% surfactant solution.

1,2-dioleoyl-sn-glycero-3-phosphoethanolamine-N-(1-pyrenesulfonyl) (ammonium salt) or Pyrene lipid linker solution - 1 mL of 1,2-dioleoyl-sn-glycero-3-phosphoethanolamine-N-(1-pyrenesulfonyl) (ammonium salt) (Avanti Lipids) provided in chloroform (1 mg/mL) was evaporated under argon and then 10 mL of 0.1% DDM solution was added to prepare the above solution.

1,2-diphytanoyl-sn-glycero-3-phosphocholine lipids (DiPhyPC) stock solution in PBS - 2mgs of DiPhyPC (Avanti Lipids) was added to 1mL of PBS buffer with gently vortexing to prepare the above stock solution.

DiPhyPC in 0.1% DDM solution - 0.5 mL of DiPhyPC (stock solution) was added to 100mL of 0.1% DDM. The solution was stirred gently till clear.

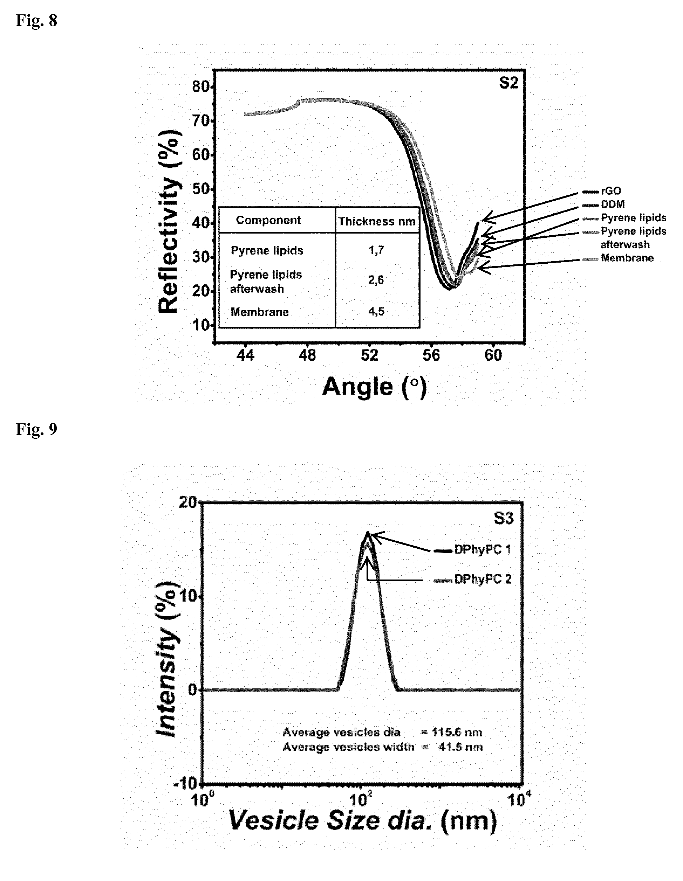

DiPhyPC vesicles in PBS solution - DiPhyPC vesicles in PBS solution were prepared as described in Keller, Biophys. J. 1998, 75, (3), 1397-1402. The size of the vesicles was measured using Dynamic light scattering technique (Malvern Zetasizer Nano-ZS) as shown in Fig. 9.

2,2'-(1-carboxy-5-(4-(pyren-1-yl)butanamido)pentylazanediyl)diacetic acid "Pyrene NTA (PyNTA) linker solution - 2mgs of 2,2'-(1-carboxy-5-(4-(pyren-1-yl)butanamido)pentylazanediyl)diacetic acid ("Pyrene NTA linker (PyNTA)) (Allichem USA) were solubilized in 10 mL PBS solution to prepare 1mM PyNTA linker solution.

## Example 14. Surface plasmon resonance (SPR) and Electro-impedance spectroscopy (EIS) procedure for observing hyb-tBLM and hyb-ptBLM formation

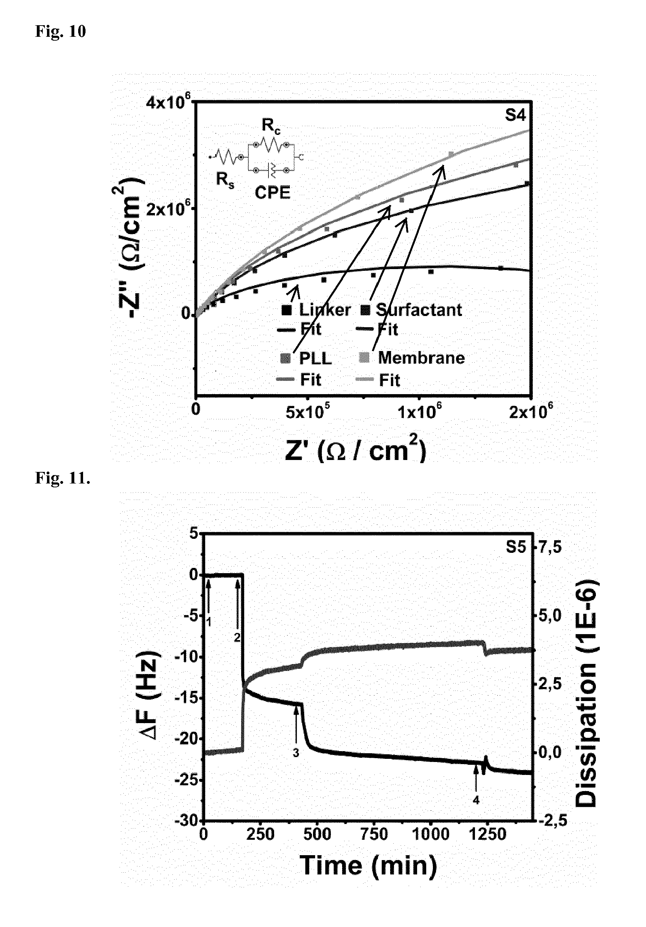

SPR (home-built) and EIS (Autolab 306 by Metrohm) were performed simultaneously on the same assembly described below. hyb-tBLM formation - The formation of hyb-tBLM was observed using SPR technique by measuring the kinetics at a fixed angle and angle scans for each layer of the assembly after their formation. Angle scan was measured on the rGO surface and then kinetic curve of the rGO surface was monitored for 1hr at $\Phi = 56°$. 4-8 mL surfactant (DDM) solution was added and the system was allowed to stabilize while performing EIS measurements after 10 minutes of DDM addition. EIS measurements were done using standard parameters defined in Giess, Biophys. J. 2004, 87, (5), 3213-3220. The above procedure was repeated after addition of each layer. Only in case of Pyrene lipids, the system was stabilized for 16 hrs while observing the kinetics and then excess pyrene lipids were washed with PBS solution to form the stable pyrene lipids layer (PLL). Impedance measurement was performed along with the angle scans. SM2-biobeads (Bio-Rad) were prepared as defined in Naumann, Langmuir 2003, 19, (13), 5435-5443. After addition of DiPhyPC and bio-beads, the system was placed undisturbed for almost 12-16 hrs while observing the kinetics. After 12-16 hrs excess lipids were washed with PBS solution followed by Impedance and angle scan measurements. The angle scans and EIS measurements are shown in Fig. 8 and Fig. 10.

hyb-ptBLM formation - The rGO modified gold surface was incubated with 1mM PyNTA linker solution for 12-16 hrs. The surface was then washed thoroughly with DI water and then incubated for 15 min in 40 mM Nickel chloride solution (NiCl$_2$.6H$_2$O). Angle scan

was measured on the PyNTA modified rGO surface and then kinetic curve of the PyNTA modified rGO surface was monitored for 1hr at $\Phi$ 56.22°. The procedure adopted for hyb-tBLM formation was repeated for each component of the hyb-ptBLM system.

## Example 15. Cyclic Voltammetry (CV) on hyb-ptBLM system

Cyclic voltammetry was performed as described in Schach, J. Electroanal. Chem. 2010, 649, (1-2), 268-276.

## Example 16. Quartz crystal microbalance with dissipation (QCM-D) procedure for observing hyb-tBLM and hyb-ptBLM formation

Quartz crystal microbalance with dissipation ( Q-Sense, QCM-D E4) was also used to study step-wise assembly of the hyb-tBLM and hyb-ptBLM platforms.

## Example 17. QCM-D chip preparation

Gold coated QCM-D chips (Q-sense) were cleaned carefully as described in literature. The chips were then functionalized with 1% cysteamine solution in ethanol for 1hour and then washed thoroughly with ethanol. The chips were dried with argon gas and the filter press method (as described above in 1 Preparation of gold surface) above was used for depositing GO onto the QCM-D chips which were further reduced. The temperature of the QCM-D cell was maintained at 25°C throughout the experiment for both systems. Buffer solution was degassed before preparing the other standard solutions for the experiment as described above in solution preparation for hyb-tBLM and hyb-ptBLM systems. All solutions were prepared freshly before each experiment. hyb-tBLM formation - Degassed PBS solution was injected into the QCM-D cell at the flow-rate of 100 $\mu$L/min for 20 min using a peristaltic pump (Ismatec IPC). The formation of hyb-tBLM was observed for each layer of the assembly by measuring frequency change ($\Delta F$) and dissipation change ($\Delta D$) at the 3rd, 5th, 7th and 9th overtones. After 3hours of stabilization of the PBS layer, 0.1% DDM solution was added slowly at flow-rate of 20 $\mu$L/min for 20 min and the system was allowed to stabilize for 3 hours before the next layer addition. The above procedure was repeated for each layer except in case of pyrene lipid layer formation the system was stabilized for 16 hrs. Excess pyrene lipids were washed by flowing PBS solution at flow-rate of 20 $\mu$L/min for 20 min to form the stable pyrene lipids layer (PLL) (Fig. 5). Lipid vesicles 0.5 mL were added to 100 mL PBS solution containing bio-beads. The vesicle solution was added to the QCM-D cell at flow-rate of 20 $\mu$L/min for 12-16 hrs to perform ex-situ dialysis. The data was fitted using Voigt model assuming layer density value of 900 kgs/m$^3$.

hyb-ptBLM formation - The rGO modified gold surface was incubated with 1 mM PyNTA linker solution for 12-16 hrs. The surface was then washed thoroughly with DI water and then incubated for 15 min in 40mM Nickel chloride solution (NiCl$_2$.6H$_2$O) and again washed thoroughly with DI water and dried with argon. The procedure used for formation of hyb-tBLM was repeated for each component of the hyb-ptBLM system.

## Example 18. Step-wise assembly of hyb-tBLM and its characterization with SPR, EIS and QCM-D techniques

The step-wise assembly of hyb-tBLM was divided into formation of the first leaflet over rGO using 1,2-dioleoyl-sn-glycero-3-phosphoethanolamine-N-(1-pyrenesulfonyl) (ammonium salt) (Pyrene lipids linker), solubilized in a non-ionic surfactant (n-Dodecyl ß-D-maltoside or DDM) followed by formation of lipid bilayer which was performed with a modified protocol using 1,2-diphytanoyl-sn-glycero-3-phosphocholine (DPhyC or lipids) vesicles and studied in detail using SPR, EIS and QCM-D monitoring techniques

## Example 19. Step-wise assembly of hyb-ptBLM and its characterization with SPR, EIS and QCM-D techniques

hyb-ptBLM was constructed with 2,2'-(1-carboxy-5-(4-(pyren-1-yl)butanamido)pentylazanediyl)diacetic acid "Pyrene NTA linker (PyNTA)". The immobilization and the formation of bilayer were studied in detail again using the three methods.

## Example 20. Step-wise assembly of hyb-tBLM and its characterization with SPR, EIS and QCM-D techniques

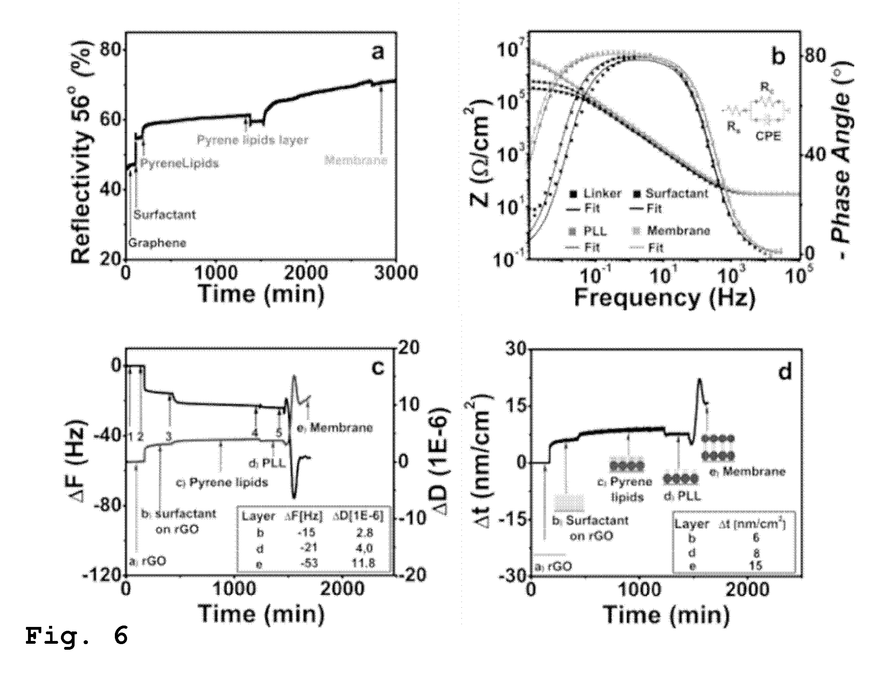

Fig. 6a and 6b depict the SPR kinetics and EIS (Bode Plots) which were done in parallel for this system. SPR showed a distinct change in refractive index as seen from the kinetic trace (Angle scan with optical thickness of layers shown in Fig. 8) upon addition of pyrene lipids linker (shown as pink squares).

We believe that the incubation process for the pyrene lipids linker leads to transfer from the surfactant phase to the reduced graphene oxide surface, due to the relatively strong n-n stacking interactions between the two non-polar aromatic rings. This aids in the formation of the first leaflet which we have defined as the "Pyrene lipid layer (PLL)" (shown in green in Fig. 6a). Rinsing with phosphate buffer solution (PBS pH 8.0) does not destabilize the layer, thus justifying our above presumption and the stability of the first leaflet. For bilayer formation, small unilamellar vesicles (SUVs) of lipids (DPhyC) with an average size 100nm (Dynamic Light Scattering data shown in Fig. 9) were added along with bio-beads to the SPR-cell. We believe that the hydrophobicity of the PLL triggers the destabilization and then fusion of vesicles to form second leaflet of the bilayer (shown as orange squares in Fig. 6a). This was also one of the widely observed mechanisms for bilayer formation in tBLM systems.

Electrochemical characterization of the membrane was simultaneously monitored using EIS method shown in Fig. 6b and Fig. 10 with three electrode system; frequency range of $10^{-3}$Hz to $10^{+5}$Hz; potential set to 0V and amplitude of the sine wave potential set at 10mV. Bode plots highlight the increase in resistance upon deposition of the PLL onto the rGO surface (shown as green squares) and the same phenomena was observed upon bilayer formation (shown as orange squares). Nyquist Plot (Fig. 10) also confirms the decrease in the charge transfer process with the solution surrounding the system during the construction of layers which can be correlated with the values in Table 3. The fits for the above two plots were defined by the electrical circuit (shown in the insert in Fig. 6b and Fig. 10). In this circuit the resistance of the individual layers of the system denoted by Rc. is coupled in parallel with the constant phase element (CPE) which defines conductivity of the layers and this system is in series with the resistance of solution surrounding the membrane (referred to as Rs). The circuit was chosen in agreement with the fit obtained for the measured values and with the EIS measurements performed by previous literature on graphene. As seen from the Table 3, there was almost a 12 fold increase in resistance upon PLL formation in comparison with rGO surface.

**Table 3.** Resistance and conductance values for layers of the hyb-tBLM system

| Layers | Rs[a] $\Omega$/cm$^2$ | Rc[b] k$\Omega$/cm$^2$ | C[c] $\mu$S/cm$^2$ | N |
|---|---|---|---|---|
| rGO | 28.4 | 287 | 22.2 | 0.89 |
| Surfactant | 30.5 | 564 | 18.5 | 0.89 |
| PLL | 19.6 | 3500 | 14.9 | 0.89 |

(continued)

| Layers | Rs[a] $\Omega$/cm$^2$ | Rc[b] k$\Omega$/cm$^2$ | C[c] $\mu$S/cm$^2$ | N |
|---|---|---|---|---|
| Membrane | 28.7 | 3890 | 14.7 | 0.90 |

[a] resistance of solution surrounding the system, [b] resistance of each layers, [c] conductance of each layer of the system.

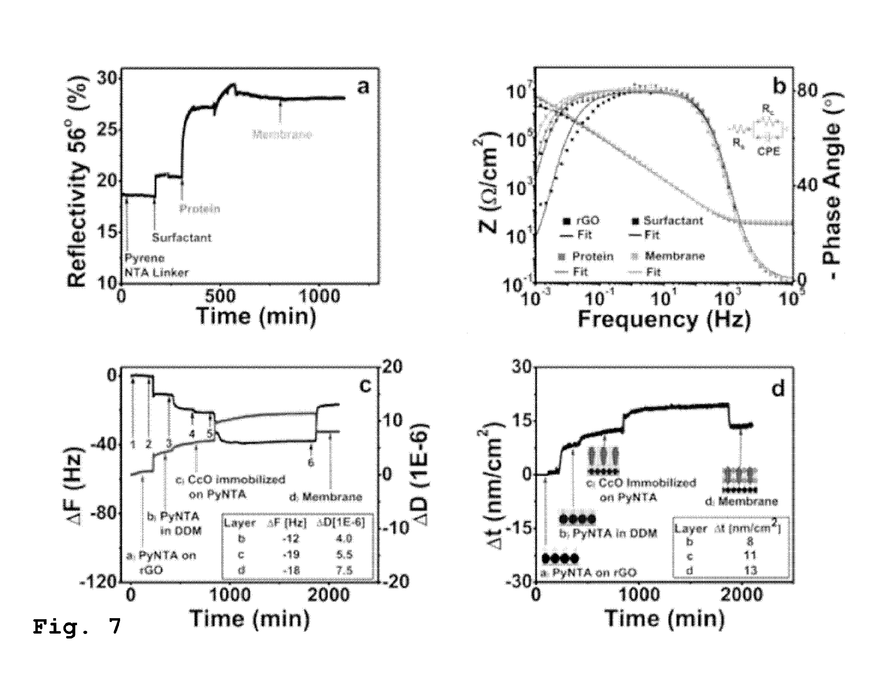

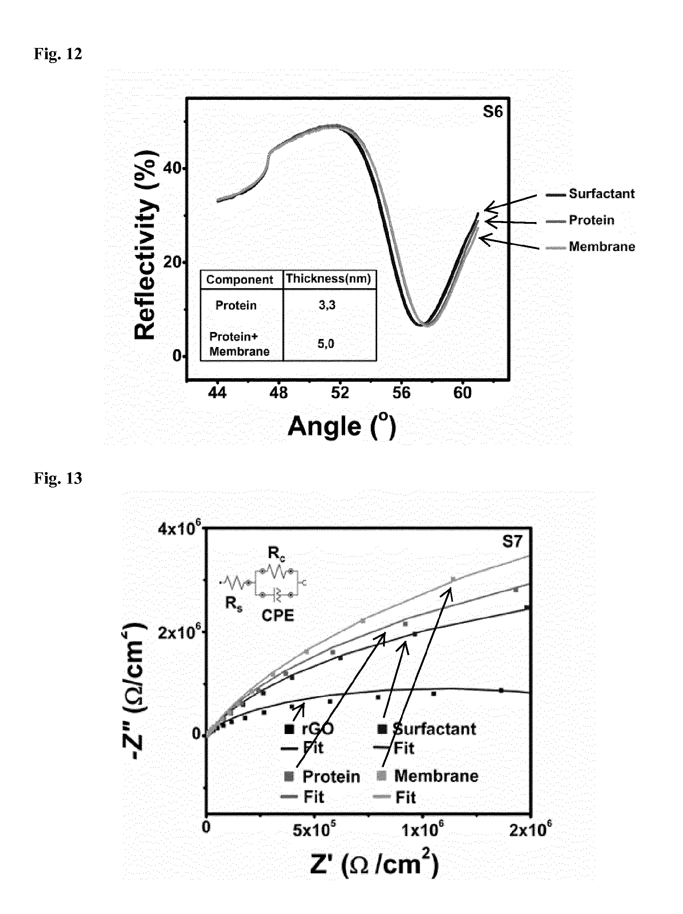

This resistance was higher than the resistance of surfactant which further supports our presumption made above regarding PLL formation and stabilization. Upon bilayer formation a 13 fold increase in resistance of the membrane was observed as compared to rGO. Bode (Fig. 6b, 7b) and Nyquist plots (Fig. 10, 13) could be collectively used to confirm the formation of the hyb-tBLM and hyb-ptBLM system along with the uniformity and sealing properties of the bilayer membrane formed. The value of N clearly signifies that the bilayer system is close to being an ideal capacitor (N=1). The relative change in resistance and capacitance values were in good agreement with values for bilayer formation. Thus we have shown for the first time formation of a stable PLL and the bilayer over reduced graphene oxide using the QCM-D technique.

The step-wise assembly was also monitored on 5MHz quartz crystal using QCM-D as shown in Fig. 6c and 6d. From QCM-D theory it is known that the frequency shift is proportional to the mass adsorbed on the surface. This can be modeled by Sauerbrey model (for rigid systems) and Voigt model (viscoelastic systems). Since membranes lack rigidity, slip over the electrode surface and show change in mass higher than the mass of the crystal, Voigt model was a good choice for calculating thickness values of each layer. Voigt model takes into account the viscoelastic properties of the membrane like density, viscosity and elasticity which can be used to calculate change in frequency and to calculate the thickness or the adsorbed mass of each layer deposited onto the QCM-D crystal. For our hyb-tBLM system, QCM-D plot (Fig. 6c) shows change in frequency '$\Delta F$' (shown in black) and change in dissipation , '$\Delta D$' (shown in red) and the thickness change '$\Delta t$' of each layer calculated from the Voigt model is as shown in Fig. 6d. Thus from the insert shown in Fig. 6c it is clear that the surfactant (ß-Dodecyl Maltoside) commonly used for solubilizing pyrene lipids linker has affinity for the rGO surface showing a corresponding $\Delta t = 6$ nm/cm$^2$. Value of $\Delta t = 8$ nm/cm$^2$ (Fig. 6d insert) observed upon stabilization of the PLL was slightly higher than the value observed in optical thickness using SPR (Fig. 8). This can be attributed to the surfactant adsorbed along with the Pyrene lipid layer. Thus QCM-D data complements the SPR and EIS results and further confirms our presumption. $\Delta t = 15$ nm/cm$^2$ upon membrane formation was again higher than SPR thickness (Fig. 8) which could be due to the adsorption of lipid vesicles over the membrane. The characteristic curve for membrane formation and values for our hyb-tBLM system (Fig. 6) correspond with literature values.

**Example 21. Step-wise assembly of hyb-ptBLM and its characterization with SPR, EIS and QCM-D techniques**

Fig. 7a summarizes the SPR measurements for hyb-ptBLM. Upon addition of CcO (shown in green) solubilized in the surfactant, it was observed that there was a sharp increase in kinetic trace confirming immobilization of the protein onto the PyNTA and Ni complex. In the final step the lipids solubilized in the detergent were added to the SPR cell with bio-beads for in-situ dialysis thus reconstituting the protein with the lipids. The trend observed in the kinetic trace corresponds with literature on ptBLMs. From Fig. 7b and Fig. 13, increasing trends in resistance values along with membrane resistance values in the M$\Omega$/cm$^2$ range corresponded with existing ptBLMs literature.

The electrical properties of the system showed approximately one order of magnitude increase in the value of membrane resistance compared to the non-covalently functionalized rGO layer (Table 4).

**Table 4.** Resistance and conductance values for layers of the hyb-ptBLM system

| Layers | Rs [a] $\Omega$/cm2 | Rc [b] M$\Omega$/cm2 | C [c] $\mu$S/cm2 | N |
|---|---|---|---|---|
| PTNTA | 26.6 | 2.2 | 14.4 | 0.89 |
| Surfactant | 26.6 | 7.7 | 13.4 | 0.89 |
| CcO | 26.5 | 10.7 | 13.0 | 0.90 |
| Membrane | 28.7 | 16.2 | 13.5 | 0.89 |

[a] resistance of solution surrounding the system, [b] resistance of each layers, [c] C - conductance of each layer of the system.

Furthermore we have demonstrated from CV measurements (Fig. 14) that the protein maintains its biological function within our hyb-ptBLM system. The characteristic peaks in the CV at -130 mV vs SHE corresponded with electron transfer from the electrode to the protein redox centers (we believe the shift in value is due to the underlying graphene layer) and at -530 mV vs SHE corresponded with reduction of protons. For the hyb-ptBLM system (Fig. 7), the peaks obtained in the CV (Fig. 15) were comparable with ptBLMs. Finally QCM-D experiments were performed on the system as shown in Fig. 7c and 7d). The protein solubilized in the detergent after addition and stabilization showed the corresponding $\Delta F$, $\Delta D$ and $\Delta t$ values of -19 Hz, 5.5 E-6 and 11 $nm/cm^2$ (surface area of QCM-D cell is approximately $1cm^2$) which was slightly higher than the actual known height of the protein (9 nm). Membrane formation corresponding to $\Delta t$ value of 13 $nm/cm^2$ was slightly higher than the SPR optical thickness value (Fig. 12) which can be caused due to the mass of entrapped water molecules which swell the lipid bilayer during the final washing step (step 6 shown in Fig. 7d). Thus we have successfully demonstrated for the first time formation of membrane formation using the QCM-D technique for the ptBLM system.

With our novel membrane architectures and the three characterization techniques we have been successful in forming a close and densely packed membrane architecture with good sealing properties on top of graphene surface, thus laying the foundation for fabrication of graphene membrane chips. These membrane chips can be used for studying interactions of ligand-membrane receptor with pharmacological or clinical relevance like ion channels, transporters and G-protein coupled receptors (GPCRs). We thus foresee our above system to create a paradigm shift in the next generation of bio-nanoelectronic sensing.

## Claims

1. A biomolecular sensor device comprising at least one graphene layer as semi-conductor material, wherein said graphene layer comprises bound to its surface a ligand binding protein, with the protein modifying an electrical property of said device, such as impedance or transistor gating of the graphene semi-conductor, especially upon ligand binding and/or conformational change of said protein, wherein said ligand binding protein is an olfactory ligand binding protein.

2. The sensor according to claim 1, with a lipid bilayer being positioned on top of said graphene layer.

3. A graphene layer with an attached lipid bilayer, wherein said graphene layer is in continuous area contact with one layer of lipids of said lipid bilayer, in particular with a hydrophilic side of the lipids of said layer of lipids.

4. The graphene layer according to claim 3, wherein said lipid bilayer comprises inserted therein at least one membrane protein, preferably wherein said membrane protein is a ligand binding protein, especially preferred an olfactory ligand binding protein.

5. A biomolecular sensor device comprising a graphene layer according to claim 3, preferably with a membrane protein according to claim 4.

6. The sensor according to claim 1, 2 or 5, being a field-effect transistor comprising a source, a gate and a drain, wherein the gate comprises as semiconductor the at least one graphene layer.

7. The sensor or graphene layer according to any one of claims 1, 2, 4 to 6, wherein said protein is a membrane protein or not a membrane protein.

8. The sensor or graphene layer according to any one of claims 1, 2, 4 to 7, wherein said protein is an odorant binding protein or an olfactory receptor.

9. The sensor or graphene layer according to any one of claims 1, 2, 4 to 8, wherein the protein is immobilized to the graphene layer by a linker molecule.

10. The sensor or graphene layer according to any one of claims 1 to 9, wherein the graphene layer comprises graphene sheets of about 500 $\mu m^2$ or more, and/or comprises grapheme sheets with an average size of between 200 $\mu m^2$ to 2000 $\mu m^2$, preferably 300 $\mu m^2$ to 1500 $\mu m^2$.

11. A method of determining the interaction of an analyte with a protein using a sensor according to any one of claims 1, 2, 5 to 10, with said protein being bound to the surface of the graphene layer of the sensor, wherein said analyte potentially interacts with said protein, whereby an electrical property of said device, such as impedance or transistor gating of the graphene semi-conductor, is modified and said modification is determined.

**12.** The method of claim 11, wherein said electrical property is electric resistance or conductivity between a source and drain electrode of a field effect transistor when a potential is applied to a gate electrode, wherein said potential is modified by said interaction of the analyte with the protein.

**13.** Method of manufacture of a graphene layer according to claim 3 or 4 comprising the step of providing a sheet of graphene on a substrate, optionally wherein said graphene is attached to said substrate with an adhesive, preferably an aminosilane, applying a lipid linker with a graphene adhesive functionality to said graphene layer, and applying lipid molecules to said graphene, preferably in form of lipid vesicles.

**14.** Method of manufacture of a sensor according to any one of claims 1, 2, 5 to 10, comprising the step of providing a sheet of graphene on a substrate, attaching a protein to said graphene layer by either i) further attaching a lipid bilayer on said graphene layer, introducing a membrane protein into said lipid bilayer, or ii) binding a linker with a graphene adhesive functionality on said graphene layer, and attaching a protein to said graphene bound linker.

**15.** Use of a sensor or graphene layer according to any one of claims 1 to 10 or as manufactured according to claim 13 or 14 in a sensor.

Fig. 1

Fig. 2

Fig. 3

Fig. 4

Fig. 5

Fig. 6

Fig. 7

**Fig. 8**

| Component | Thickness nm |
|---|---|
| Pyrene lipids | 1,7 |
| Pyrene lipids afterwash | 2,6 |
| Membrane | 4,5 |

**Fig. 9**

Average vesicles dia = 115.6 nm
Average vesicles width = 41.5 nm

**Fig. 10**

**Fig. 11.**

**Fig. 12**

**Fig. 13**

**Fig. 14**

**Fig. 15**

## EUROPEAN SEARCH REPORT

Application Number

EP 13 18 4003

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/085715 A1 (UNIV PENNSYLVANIA [US]) 13 June 2013 (2013-06-13) | 1,6-9, 11,12, 14,15 | INV. G01N27/414 G01N33/543 G01N33/00 |
| Y | * paragraphs [0024] - [0043] * | 10 | |
| X | WO 2011/082178 A1 (NANOSENSE INC [US]; RHODES PAUL A [US]; KHAMIS SAMUEL M [US]) 7 July 2011 (2011-07-07) * abstract * * paragraphs [0008], [0025] - [0026], [0032] - [0034], [0037], [0039]; figure 1 * | 1-8,11, 12,14,15 | |
| X | US 2011/059544 A1 (HONG SEUNG-HUN [KR] ET AL) 10 March 2011 (2011-03-10) * paragraphs [0046] - [0053], [0063], [0070] - [0073]; figures 1-5 * | 1-8,11, 12,14,15 | |
| Y,D | MELANIE LARISIKA ET AL: "An improved synthesis route to graphene for molecular sensor applications", MATERIALS CHEMISTRY AND PHYSICS, vol. 136, no. 2-3, 1 October 2012 (2012-10-01), pages 304-308, XP055100047, ISSN: 0254-0584, DOI: 10.1016/j.matchemphys.2012.08.003 * abstract * * page 306, column 2, paragraph 1 * * page 307, column 2, paragraph 4 - page 308, column 1, paragraph 4 * | 10 | TECHNICAL FIELDS SEARCHED (IPC) G01N |

-/--

The present search report has been drawn up for all claims

| Place of search | Date of completion of the search | Examiner |
|---|---|---|
| Munich | 5 February 2014 | Lazar, Zala |

EPO FORM 1503 03.82 (P04C01)

Europäisches
Patentamt

European
Patent Office

Office européen
des brevets

## EUROPEAN SEARCH REPORT

Application Number

EP 13 18 4003

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OKAMOTO Y ET AL: "Fabrication of Supported Lipid Bilayer on Graphene Oxide", JOURNAL OF PHYSICS: CONFERENCE SERIES, INSTITUTE OF PHYSICS PUBLISHING, BRISTOL, GB, vol. 352, no. 1, 5 March 2012 (2012-03-05) , page 12017, XP020220216, ISSN: 1742-6596, DOI: 10.1088/1742-6596/352/1/012017 * abstract * * page 2, paragraph 2-3 * * page 4, paragraph 2 - page 5, paragraph 1; figure 4 * | 3,4,7,8, 13,15 | |
| A | US 2012/214172 A1 (CHEN JUNHONG [US] ET AL) 23 August 2012 (2012-08-23) * paragraphs [0037] - [0045]; figure 1 * | 1-15 | |

TECHNICAL FIELDS
SEARCHED       (IPC)

The present search report has been drawn up for all claims

| Place of search | Date of completion of the search | Examiner |
|---|---|---|
| Munich | 5 February 2014 | Lazar, Zala |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 13 18 4003

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2014

| Patent document cited in search report | | Publication date | Patent family member(s) | | Publication date |
|---|---|---|---|---|---|
| WO 2013085715 | A1 | 13-06-2013 | NONE | | |
| WO 2011082178 | A1 | 07-07-2011 | US | 2012028820 A1 | 02-02-2012 |
| | | | WO | 2011082178 A1 | 07-07-2011 |
| US 2011059544 | A1 | 10-03-2011 | KR | 20090116652 A | 11-11-2009 |
| | | | US | 2011059544 A1 | 10-03-2011 |
| US 2012214172 | A1 | 23-08-2012 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1233262 A1
- US 2003017618 A1
- US 6598459 B1
- US 6844197 B1
- US 2012028820 A1
- CA 2666662 C
- US 2010188110 A1
- US 2012021932 A1
- GB 2485559 A
- JP 2012247189 A
- US 2011027853 A
- US 2012220053 A
- US 2013018599 A1
- WO 2009035647 A1
- WO 2010059687 A2
- WO 2010097518 A1
- WO 2012150884 A1
- WO 2012005857 A1
- WO 2012050646 A2
- US 20130018599 A

**Non-patent literature cited in the description**

- **MISRA et al.** *Applied Physics Letters,* 2007, vol. 90, 052905
- **WOGULIS et al.** *Biochemical and Biophysical Research Communications,* 2006, vol. 339 (1), 157-164
- **GAILLARD et al.** *CMLS, Cell. Mol. Life Sci.,* 2004, vol. 61, 456-469
- **DANI et al.** *J Proteome Res,* 2010, vol. 9 (4), 1822-1833
- **IOVINELLA et al.** *Journal of Proteome Research,* 2011, vol. 10 (8), 3439-3449
- **LARISIKA et al.** *Materials Chemistry and Physics,* 2012, vol. 136 (2-3), 304-308
- **LANGMUIR.** *Journal of the American Chemical Society,* 1918, vol. 40 (9), 1361-1403
- **KELLER.** *Biophys. J.,* 1988, vol. 75 (3), 1397-1402
- **GIESS.** *Biophys. J.,* 2004, vol. 87 (5), 3213-3220
- **NAUMANN.** *Langmuir,* 2003, vol. 19 (13), 5435-5443
- **SCHACH, J.** *Electroanal. Chem.,* 2010, vol. 649 (1-2), 268-276